Europäisches Patentamt

European Patent Office

Office européen des brevets

(11) Publication number: **0 246 999**
**A1**

# (12) EUROPEAN PATENT APPLICATION

(21) Application number: **87630094.8**

(22) Date of filing: **19.05.87**

(51) Int. Cl.4: **A 61 F 5/00**

(30) Priority: **23.05.86 IL 78883**

(43) Date of publication of application:
**25.11.87 Bulletin 87/48**

(84) Designated Contracting States:
**AT BE CH DE ES FR GB IT LI NL SE**

(71) Applicant: **OMIKRON SCIENTIFIC LTD.**
**Kiryat Weizmann**
**Rehovot 76120 (IL)**

(72) Inventor: **Eshel, Uzi**
**28 Ahi-Dakar St.**
**Herzlia (IL)**

**Lev, Avigdor**
**12 Mordechai Chefetz St.**
**Petach Tikva (IL)**

**Gertller, Aharon**
**35 Weizmann St.**
**Natanya (IL)**

(74) Representative: **Schmitz, Jean-Marie et al**
**OFFICE DENNEMEYER S.à.r.l. P.O. Box 1502**
**L-1015 Luxembourg (LU)**

(54) **Device and method of making same for treating a patient for obesity.**

(57) A device for insertion into the stomach of a patient for treating the patient for obesity, comprises an inflatable balloon (2) including a self-closing valve (23) at one end (21) for intrducing an inflating fluid, and a projecting ear (24) integrally formed with the balloon at the opposite end (22) for engagement by forceps in order to push the respective end (22) of the balloon (2), and thereby to pull the complete balloon (2), via the esophagus into the patient's stomach. Also described is a method and apparatus making the balloon (2) so as to have a smooth seamless surface.

FIG. 2

## Description

DEVICE AND METHOD OF MAKING SAME FOR TREATING A PATIENT FOR OBESITY

The present invention relates to a device and method for treating a patient for obesity, and particularly to an inflatable balloon device which is introduced into the stomach for this purpose.

In the past few years, it has been found that extreme obesity can be successfully treated by introducing a balloon into the patient's stomach and inflating the balloon with a liquid, air or other gas. The balloon decreases the volume of the stomach so that the feeling of satisfaction and fullness occurs after very little food has been consumed by the patient. This technique for treating obesity has been described in many publications, including U.S.A. Patents 4,416,257 and 4,485,805; European Patent Application 0137878, and British Patent Application 2,139,902a.

An object of the present invention is to provide provide a device to be inserted into the stomach of a patient for treating the patient for obesity, having advantages over the known techniques in a number of respects as will be described more particularly below. Another object of the invention is to provide a method of making the balloon used in this device.

According to one aspect of the present invention, is provided a device for insertion into the stomach of a patient for treating the patient for obesity, comprising: an inflatable balloon including a self-closing valve at one end for introducing an inflating fluid, and a projecting ear integrally formed with the balloon at the opposite end for engagement by rigid forceps in order to push the respective end of the balloon, and thereby to pull the complete balloon, via the esophagus into the patient's stomach.

According to important features in the preferred embodiment of the invention described below, the balloon ear is apertured for receiving the pin of the forceps when inserting the balloon into the patient's stomach; also, the self-closing valve is of tubular configuration and is adhesively bonded to the inside face of the balloon, the balloon having been pierced at the respective end at a point in alignment with the self closing-valve.

According to another aspect of the invention, there is provided a method of making the above balloon for introduction into the stomach of the patient in order to treat the patient for obesity, comprising: forming the wall of the balloon by dip coating an elastomeric material on a mould such that one end of the wall is closed and the opposite end is open; turning the so formed balloon wall inside out; adhesively securing a self-closing valve to the exposed face of the balloon wall at the closed end thereof; turning the balloon wall back to its original condition; piercing the hole in alignment with the valve; and pinching closed the open end of the balloon wall to produce the ear for use in introducing the balloon into the patient's stomach.

The invention provides a number of advantages over existing techniques. Thus, the technique for inserting the balloon is extremely simple to perform and can be done very quickly, as compared to prior techniques. Further, the gastroscope can be used for visual observation during the insertion of the balloon into the stomach and during its manipulation to properly locate it in the stomach. The balloon is constructed to have a smooth flush surface with no seams thus protecting the lining of the stomach; and the technique provides more softness and minimum inconvenience to the patient.

Further features and advantages of the invention will be obtained from the description below.

The invention is herein described, by way of example only, with reference to the accompanying drawings wherein;

Fig. 1 illustrates the device of the present invention at the time of its insertion into the stomach of the patient for treating the patient for obesity;

Fig. 2 illustrates the construction of the balloon in its inflated condition;

Fig. 3 is a fragmentary sectional view along lines III-III of Fig. 2;

Fig. 4 is a view similar to that of Fig. 3 but illustrating a modification; and

Fig. 5 is a sectional view illustrating the structure of the filling tube and its blunt, rigid tubular nozzle at the end received within the self-closing valve of the balloon for inflating the balloon.

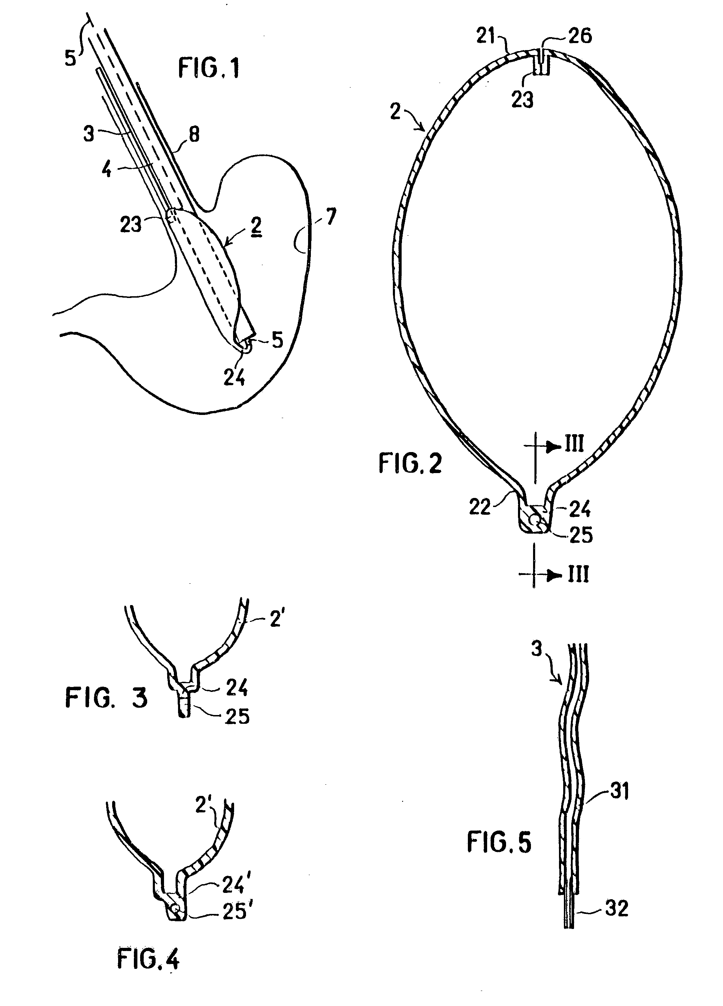

Fig. 1 illustrates the main components of the device which are inserted into the patient's stomach in order to treat the patient for obesity. These components include a balloon, generally designated 2, a filling tube 3 for use in inflating the balloon after it has been placed in the patient's stomach; a gastroscope 4 providing visual observation during the insertion of the balloon and its proper placement in the stomach; and forceps 5 passed through the gastroscope 4 and engageable with an ear formed at one end of the balloon for introducing the balloon into the stomach. All the foregoing components are introduced with the balloon into the stomach 7 by manipulating the forceps 5 to grasp the balloon ear at the lower end of the balloon and then pushing the balloon ear through the esophagus, while the filling tube 3 is attached to the opposite end of the balloon and while the balloon is wrapped around the outer face of the gastroscope 4. The actual technique for insertion of the balloon via the patient's esophagus 8 into the patient's stomach 7 is more particularly described below.

The structure of balloon 2 is illustrated in Fig. 2. It is of oblong shape, being of smaller diameter at its opposite ends 21, 22. End 21 carries a self-closing valve 23 of the type commonly used for inflating balls, such as basketballs or footballs, except that it is not carried by a patch, as in the case with inflatable balls, but rather is adhesively bonded to the inner face of the balloon wall at its closed end 21. In addition, self-closing valve 23 is of smaller diameter than the conventional valves used for inflating balls so as to minimize the dimensions of the balloon when inserted in deflated condition through the esophagus into the patient's stomach. The balloon is then pierced at point 26 in alignment with the mouth of valve 23.

The opposite closed end 22 of the balloon 2 is formed with an ear 24 having an aperture 25 therethrough. Ear 24 is grasped by the forceps 5, passing through the gastroscope 4, when introducing the balloon into the patient's stomach. The forceps used may be one of the known flat types. In this case, however, it preferably includes a pin (not shown) at its grasping end for passing through aperture 25 in ear 24, in order to better grip the ear when pushing it through the esophagus 8 and into the stomach 7.

Ear 24 may be of uniform width and thickness, as shown in Fig. 3, with aperture 25 passing centrally through the ear. An alternative arrangement is illustrated in Fig. 4, wherein ear 24' formed at the respective end of balloon 2' is of non-uniform width, being narrower at one end wherein its aperture 25 is formed, to provide better observation when introducing the balloon via the esophagus into the patient's stomach. An alternative arrangement is to tie a short suture to the ear and to grasp the suture with the forceps, thus also enabling better viewing.

Gastroscope 4 may be of a conventional type presently used by passing through the mouth and the esophagus into the patient's stomach in order to view the interior of the patient's stomach and to remove objects therefrom. It is of hollow construction to permit the insertion of the forceps 5 therethrough.

Filling tube 3, more particularly illustrated in Fig. 5, comprises a flexible hollow tube 31 having a rigid, blunt tubular nozzle 32 secured at one end. Nozzle 32 is inserted into the self-closing valve 23 through hole 26 in the balloon 2 before both are introduced into the patient's stomach. After the balloon has been inflated, by liquid or air via the filling tube 32, the filling tube is pulled outwardly first to remove the nozzle from the valve 23, and then to pull the nozzle out through the patient's mouth. Since nozzle 32 is blunt (not sharp), there is little danger of damaging the patient's esophagus when it is removed in this manner.

Balloon 2 may be formed of any suitable elastomeric material, such as silicone rubber, synthetic elastomers and the like in single or multiple layers. Following is one example of producing the balloon:

First, the wall of the balloon is formed by dip coating on a mould, such that end 22, to eventually form the ear 24, is left open while end 21, to receive the self-closing valve 23, is closed. The so formed balloon wall is then turned inside out, and the self-closing valve 23 is adhesively secured to the thus exposed face of the balloon wall at its end 21.

The balloon wall is then turned back to its original condition, and pierced at point 26 in alignment with valve 23. The open end of the balloon wall is pinched closed with adhesive so as to form ear 24. Aperture 25 is then formed through ear 24.

The above method produces a balloon which includes no seams and is completely smooth on its outer face including the location of the self-closing valve 23. This construction thereby facilitates the insertion of the balloon via the esophagus into the stomach with a minimum of irritation or inconvenience to the patient.

The balloon is inserted into the patient's stomach according to the following sequence: First, nozzle 32 of filling tube 3 is inserted into valve 23 through hole 26 in balloon 2. Then, forceps 5 are passed through the gastroscope 4 to grasp ear 24 at the opposite end of the balloon. The balloon 2 is then wrapped around the gastroscope 4, and is tensioned slightly in the longitudinal direction by pushing down slightly on forceps 5. The gastroscope 4, with the balloon 2 wrapped around it and with its ear 24 grasped by forceps 5 passing through the gastroscope 4, is now inserted into the patient's mouth and pushed down through the esophagus 8 and into the stomach 7. The attendant may continuously view this operation via the gastroscope 4. Once the balloon is within the patient's stomach, it may be manipulated to be properly located within the stomach while still under observation by the attendant via the gastroscope.

When the balloon is properly located in the stomach, it is inflated by passing a fluid, e.g., a liquid, air or other gas via filling tube 3 and its nozzle 32 received within valve 23. When the balloon has been properly inflated, again under visual observation via gastroscope 4, filling tube 3 is pulled to remove its nozzle 32 from valve 23 and is then pulled out through the esophagus and the patient's mouth, while forceps 5 still grasp ear 24 of the balloon. When the filling tube has thus been removed, the forceps 5 are opened to disengage from ear 24 of the balloon, and the forceps and gastroscope are then removed via the patient's esophagus, leaving the inflated balloon within the patient's stomach. As indicated earlier, the above-described technique permits the balloon to be inserted in a very simple and quick manner and with a minimum of irritation or inconvenience to the patient. In addition, it permits the complete operation to be made under visual observation by viewing through the gastroscope.

**Claims**

1. A device for insertion into the stomach of a patient for treating the patient for obesity, comprising: an inflatable balloon including a self-closing valve at one end for intrducing an inflating fluid, and a projecting ear integrally formed with the balloon at the opposite end for engagement by forceps in order to push the respective end of the balloon, and thereby to pull the complete balloon, via the esophagus into the patient's stomach.

2. The device according to Claim 1, wherein said ear is apertured for receiving the pin of the forceps when inserting the balloon into the patient's stomach.

3. The device according either of Claims 1 or 2, further including a suture secured to said ear for grasping by the forceps in order to enable better viewing.

4. The device according to any one of Claims 1-3, wherein said self-closing valve is of tubular configuration and is adhesively bonded to the inside face of the balloon at said one end thereof, said balloon being pierced at said one end at a point in alighnment with said self-closing valve.

5. The device according to any one of Claims 1-4, wherein said balloon is of oblong shape, having a smaller curvature at its opposite ends containing the self-closing valve and projecting ear.

6. The device according to any one of Claims 1-5, further including a flexible filling tube having a rigid, blunt stem at one end received in said self-closing valve of the balloon when the balloon is introduced into the stomach, said stem being removable from the self-closing valve when the balloon is in the patient's stomach by pulling on said filling tube.

7. The device according to Claim 6, further including a hollow gastroscope inserted in the patient's stomach and permitting view thereof, and flat forceps received through the hollow gastroscope for grasping said ear of the balloon in order to push it into the stomach.

8. A method of making the balloon of Claim 1 for introduction into the stomach of the patient in order to treat the patient for obesity, comprising; forming the wall of the balloon by dip coating an elastomeric material on a mould such that one end of the wall is closed and the opposite end is open; turning the so formed balloon wall inside out; adhesively securing a self-closing valve to the exposed face of the balloon wall at said closed end thereof; turning the balloon wall back to its original condition; then piercing a hole in the balloon in alignment with the valve; and pinching closed the open end of the balloon wall to produce said ear for use in introducing the balloon into the patient's stomach.

9. The method according to Claim 8, including the further step of forming an aperture in said ear to facilitate its introduction into the patient's stomach by the use of forceps having a pin received in said aperture.

FIG.1

FIG.2

FIG.3

FIG.4

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 485 805 (FOSTER) <br> * Whole document * | 1 | A 61 F 5/00 |
| Y | US-A-4 315 509 (SMIT) <br> * Abstract; column 4, lines 41-53; figures * | 1 | |
| D,A | GB-A-2 139 902 (CELESTIN) | | |
| D,A | EP-A-0 137 878 (GARREN) | | |

TECHNICAL FIELDS SEARCHED (Int. Cl.4)

A 61 F
A 61 B

The present search report has been drawn up for all claims

| Place of search | Date of completion of the search | Examiner |
|---|---|---|
| THE HAGUE | 21-08-1987 | STEENBAKKER J. |